(19) Europäisches Patentamt / European Patent Office / Office européen des brevets

(11) **EP 3 705 141 B1**

(12) **EUROPEAN PATENT SPECIFICATION**

(45) Date of publication and mention
of the grant of the patent:
**01.10.2025 Bulletin 2025/40**

(21) Application number: **18872781.2**

(22) Date of filing: **24.10.2018**

(51) International Patent Classification (IPC):
**A61L 27/36** (2006.01)   **A61L 27/50** (2006.01)
**C07K 14/78** (2006.01)

(52) Cooperative Patent Classification (CPC):
**A61L 27/3604; A61L 27/362; A61L 27/3625;
A61L 27/3683; A61L 27/3691; A61L 27/50;
A61L 27/507;** A61L 2430/40

(86) International application number:
**PCT/JP2018/039426**

(87) International publication number:
**WO 2019/087880 (09.05.2019 Gazette 2019/19)**

(54) **SHEET-LIKE DECELLULARIZED MATERIAL AND ARTIFICIAL BLOOD VESSEL EMPLOYING SAID MATERIAL**

BLATTFÖRMIGES DEZELLULARISIERTES MATERIAL UND KÜNSTLICHES BLUTGEFÄSS MIT VERWENDUNG DES BESAGTEN MATERIALS

MATIÈRE DÉCELLULARISÉE DE TYPE FEUILLE ET VAISSEAU SANGUIN ARTIFICIEL L'UTILISANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.10.2017 JP 2017210576**

(43) Date of publication of application:
**09.09.2020 Bulletin 2020/37**

(73) Proprietor: **Adeka Corporation
Tokyo 116-8554 (JP)**

(72) Inventors:
• **YAMAGUCHI, Yu**
**Tokyo 116-8554 (JP)**
• **KIMURA, Takuya**
**Tokyo 116-8554 (JP)**

(74) Representative: **van Dam, Vincent
Octrooibureau Vriesendorp & Gaade B.V.
Koninginnegracht 19
2514 AB Den Haag (NL)**

(56) References cited:
EP-A1- 2 944 330       EP-A1- 3 305 248
WO-A1-2005/063316   WO-A1-2014/034759
WO-A1-2014/109185   WO-A1-2016/136633
WO-A1-2016/136633   WO-A1-2016/194895
WO-A1-2016/194895

• E PASQUINO ET AL: "Bovine pericardium for heart valve bioprostheses: in vitro and in vivo characterization of new chemical treatments", JOURNAL OF MATERIALS SCIENCE: MATERIALS IN MEDICINE, vol. 5, 1 January 1994 (1994-01-01), pages 850 - 854, XP055613794

• JAHNAVI S ET AL: "Engineering of a polymer layered bio-hybrid heart valve scaffold", MATERIALS SCIENCE AND ENGINEERING C, vol. 51, 1 June 2015 (2015-06-01), pages 263 - 273, XP029124734, ISSN: 0928-4931, DOI: 10.1016/J.MSEC.2015.03.009

• PASQUINO, E ET AL.: "Bovine pericardium for heart valve bioprostheses: in vitro and in vivo characterization of new chemical treatments", JOURNAL OF MATERIALS SCIENCE: MATERIALS IN MEDICINE, vol. 5, 1994, pages 850 - 854, XP055613794, ISSN: 0957-4530

- JAHNAVU, S ET AL.: "Engineering of a polymer bio-hybrid heart valve scaffold", MATERIALS SCIENCE AND ENGINEERING C, vol. 51, 2015, pages 263 - 273, XP029124734, ISSN: 0298-4931, doi:10.1016/j.msec.2015.03.009
- HOGANSON, DH ET AL.: "The retention of extracellular matrix proteins and angiogenic and mitogenic cytokines in a decellularized porcine dermis", BIOMATERIALS, vol. 31, no. 26, 2010, pages 6730 - 6737, XP009138805, ISSN: 0142-9612, DOI: 10.1016/j.biomaterials.2010.05.019

## Description

TECHNICAL FIELD

The present invention relates to an artificial blood vessel, a graft of a tubular biological tissue or a blood vessel repairing material as defined in claim 1.

BACKGROUND ART

Blood vessel grafts are used in the construction of blood vessels for bypass surgery, and in repair or replacement of damaged or morbid blood vessels. For example, in the treatment of atherosclerosis of coronary arteries or peripheral blood vessels, patients' own blood vessels are preferable replacement grafts for affected areas having a diameter less than 5 mm, and patient's own internal thoracic artery, radial artery, saphenous vein, etc. are used. However, invasive collection cannot be avoided when using patient's own blood vessel, which accordingly translates into a significant burden on the patient's body and into inevitable variability in the length and quality of the blood vessel depending on individuals or cases. Furthermore, there is a problem that in cases of reoperation, it is impossible to obtain again patients' own blood vessels as they are already in use.

An artificial blood vessel made from a synthetic resin such as polyester and polytetrafluoroethylene is used for revascularization of limb peripheral arteries and the like. However, an artificial blood vessel made from such synthetic resin cannot be used because of early thrombus formation and intima thickening, in a case where the artificial blood vessel is used in a small-diameter blood vessel such as the coronary arteries. In order to prevent blood clotting in an artificial blood vessel made from such synthetic resin, the lumen of the artificial blood vessel is covered with the patient's own vascular endothelial cells by means of tissue engineering techniques. However, the bone marrow must be collected from the patient, and be cultured and allowed to graft onto the artificial blood vessel, prior to surgery, which requires preparations in advance. The usefulness of this approach is low in surgery that must be performed in an emergency. In addition, there is also a problem that the vascular endothelial cells covering the lumen of the blood vessel are easily peeled off, which may cause thrombus formation.

Among them, Patent Literature 1 proposes an artificial blood vessel in which a sheet of decellularized biomaterial is formed into a tubular shape in order to prevent rejection. Specifically, the artificial blood vessel is formed by rolling a sheet prepared from a porcine aorta into a tubular shape without any modification. However, the edge portion of the sheet juts into the lumen of the tube, in the luminal cross-section, by the extent of the thickness of the sheet, and the cross-section of the tube does not become circular or elliptical (see: FIG. 8 in Patent Literature 1). When the cross-section of the tubular structure is in such a shape, localized pressure acts on the portion jutting out when blood flows therethrough, and as a result, peel-off may occur, and thus the pressure resistance of the blood vessel is insufficient. In addition, there arises a problem that the handleability becomes worse during surgery, if the sheet is made thicker in order to secure pressure resistance. Moreover, it is also considered that platelets and the like more easily adhere to the portion jutting out, and thrombus are more easily generated.

The invention described in Patent Literature 2 by the present applicant, is one which solves the above problem of Patent Literature 1. Patent Literature 2 describes a sheet of biological tissue, the sheet including on at least one side tapered edge portion thinning down in the thickness direction towards an end thereof, and a tubular structure using the sheet. A porcine aorta is used for preparing sheets, etc. in the examples, as in Patent Literature 1.

As mentioned above, sheets prepared from a porcine aorta are specifically used as a sheet of the biological tissue in Patent Literatures 1 and 2. Therefore, there has been a demand for a material which has more improved pressure resistance than a sheet of biological tissue derived from a porcine aorta, and which can maintain excellent pressure resistance when used as an artificial blood vessel or for repair of a blood vessel.

WO 2016/194895 A1 discloses a sheet of biological tissue, for obtaining a tubular structure having excellent pressure resistance and handleability, the sheet being usable as an artificial blood vessel.

WO 2014/109185 A1 discloses an artificial blood vessel prepared from a decellularized tubular structure, which is prepared by processing a decellularized, sheet-like blood vessel (decellularized blood vessel sheet) into a roll structure, and a tissue adhesive, wherein a portion which is contacted with blood that flows within the artificial blood vessel consists of the tissue of the tunica intima lined with the tissue of the tunica media whereas a portion of the sheet that overlaps when the sheet is processed into a roll structure (overlap width) consists of the tissue of the tunica media and wherein a tissue adhesive is applied to the overlap width.

WO 2016/136633 A1 discloses a decellularized tissue prepared by decellularizing a biological tissue, said decellularized tissue being characterized in that the ratio of endothermic energy amount, in terms of dry mass, of the decellularized tissue to the biological tissue before the decellularization is 0.70 or greater when measured by differential scanning calorimetry.

E. Pasquino et al, Journal of Materials Science: Materials in Medicine, vol.5, 1 January 1994, pages 850-854 discloses in vitro and in vivo characterization of new chemical treatments of bovine pericardium for heart valve bioprostheses:
S. Jahnavi et al, Materials Science and Engineering, vol. 51, 1 June 2015, pages 263-273 discloses engineering of a polymer layered bio-hybrid heart scaffold.

WO 2014/034759 A1 discloses a method for decellularizing a tissue using an inexpensive and compact pressure device, said method being characterized in that a high hydrostatic pressure of about 200 MPa is applied to a collected tissue while immersing the tissue in an aqueous solution to thereby decellularize the tissue.

CITATION LIST

PATENT LITERATURE

Patent Literature 1: WO 2014/109185
Patent Literature 2: WO 2016/194895

SUMMARY OF THE INVENTION

TECHNICAL PROBLEM

The problem to be solved by the present invention is to provide a sheet-like material capable of maintaining excellent pressure resistance when used as an artificial blood vessel or for repair of a blood vessel.

SOLUTION TO THE PROBLEM

As a result of intensive studies to solve the above problems, the present inventors have surprisingly found that an artificial blood vessel having a much higher pressure resistance than one derived from a porcine aorta, which was conventionally considered to be optimum, can be prepared by preparing an artificial blood vessel using a biomaterial-derived sheet-like decellularized material having a tensile strength and an elongation rate in a specific range. Thus, the present inventors have completed the present invention.
Namely, the present invention is as defined in claim 1

ADVANTAGEOUS EFFECTS OF THE INVENTION

The biomaterial-derived sheet-like decellularized material as defined in accordance with the present invention can provide an artificial blood vessel having a much higher pressure resistance strength than one derived from a porcine aorta, which was conventionally considered to be optimum. Therefore, when the biomaterial-derived sheet-like decellularized material as defined in accordance with the present invention is used as an artificial blood vessel or for repair of a blood vessel, excellent pressure resistance comparable to that of a blood vessel itself can be maintained.

BRIEF DESCRIPTION OF THE DRAWINGS

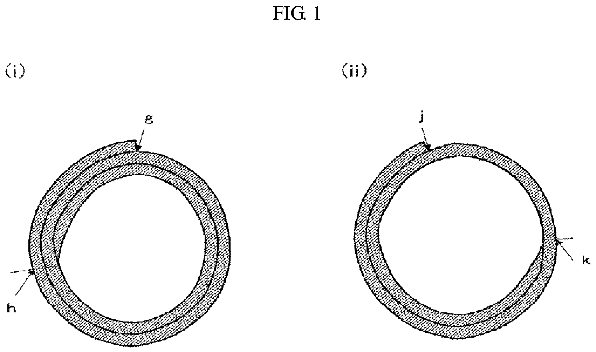

FIG. 1 (i) is a diagram illustrating a front cross-section of an artificial blood vessel in one embodiment of the present invention. FIG. 1 (ii) is a diagram illustrating a front cross-section of an artificial blood vessel in another embodiment of the present invention.

DESCRIPTION OF THE INVENTION

1. Biomaterial-derived sheet-like decellularized material

The biomaterial-derived sheet-like decellularized material as defined in accordance with the present invention is a biomaterial-derived sheet-like decellularized material having a maximum value of tensile strength in four directions of 4 MPa or more and an elongation rate in the direction exhibiting the maximum tensile strength of 50% to 300%.
A biomaterial used for the biomaterial-derived sheet-like decellularized material as defined in accordance with the present invention is an animal-derived material. The animal is preferably a vertebrate or the like, and more preferably a mammal, a bird or the like due to milder rejection reactions. A mammalian livestock, avian livestock, human or the like is more preferably used because of easy availability. Examples of the mammalian livestock include a cow, pig, sheep, horse, goat, deer, dog, cat, rabbit, hamster, guinea pig, rat, mouse, camel, llama, donkey, yak, alpaca, raccoon dog, weasel, fox, squirrel, raccoon and the like. Examples of the avian livestock include a chicken, duck, turkey, goose, guinea fowl, pheasant, ostrich, quail, parakeet, parrot and the like. Preferable animals include a pig, cow, horse, human and the like, and more preferably a pig, cow and the like in terms of availability and safety.

The site of animal tissue used as a biomaterial is pericardium, When a skin, dermis, or the like is used, the thickness of the material is large so that the handleability is poor. When a part derived from pores remains, platelets and the like easily adhere to the part and there is a problem that thrombus are easily generated. In consideration of these issues, and from the viewpoints of easiness and availability of decellularization and pressure resistance and handleability when rolled, pericardium is chosen. In addition, pericardium is chosen because excellent pressure resistance can be exhibited. Further, a pericardium is chosen because it exhibits excellent pressure resistance comparable to that of a blood vessel itself.

The biomaterial is subsequently subjected to decellularization and sheeting to prepare a biomaterial-derived sheet-like decellularized material. Decellularization may be performed before or after sheeting, or may be performed after rolling into an artificial blood vessel. However, in view of processability and easiness of decellularization, decellularization is preferably performed before rolling, and is preferably performed after forming into a sheet shape.

Decellularization is performed to remove antigenic components such as cells and nucleic acid components from biomaterials collected from an animal. By performing decellularization, it is possible to suppress the rejection that occurs when used as a transplant tissue in a living body.

The decellularization method is not particularly limited in the present invention, and a conventional known method may be used. Examples of decellularization include a physical agitation, ultrasonic processing, freeze-thaw method, high hydrostatic pressure method, hypertonic solution/hypotonic solution method, a treatment using a surfactant such as an anionic surfactant and nonionic surfactant, an enzymatic treatment by a proteolytic enzyme, nucleolytic enzyme and the like, as well as a treatment using an alcohol solvent. The foregoing may be implemented in combination of two or more thereof.

A method including a high hydrostatic pressure method is preferably used in the present invention from the viewpoint of efficient decellularization while maintaining the mechanical strength of the structural protein and from the viewpoint of blood compatibility.

In forming into a sheet, the shape of the sheet is not limited, but is preferably rectangular (oblong) or substantially rectangular, from the viewpoint of the pressure resistance of the rolled artificial blood vessel, handleability and processability. In the case of a rectangular or substantially rectangular sheet, the size of the sheet may be appropriately selected depending on the size of the desired artificial blood vessel. The length of one side of the sheet in the length direction ("long side") is usually 10 to 400 mm, preferably 20 to 300 mm, from the viewpoint of the pressure resistance and handleability when rolled. The length of one side of the sheet in the width direction ("short side") is usually 1.5 to 200 mm, preferably 3 to 70 mm, and more preferably 6 to 40 mm.

In the biomaterial-derived sheet-like decellularized material as defined in accordance with the present invention, the maximum value of the tensile strength in four directions is 4 MPa or more, preferably 5 MPa or more. By using the biomaterial-derived sheet-like decellularized material as defined in accordance with the present invention having a maximum value of tensile strength in four directions of 4 MPa or more, an artificial blood vessel prepared by rolling the same has extremely high pressure resistance strength. On the other hand, as understood from Test Examples 3 and 4, in the biomaterial-derived sheet-like decellularized material prepared using a porcine aorta or bovine aorta, the maximum values of the tensile strength in four directions are respectively 3.1 MPa and 3.9 MPa, so that the pressure resistance strengths of the artificial blood vessels prepared by rolling the same are inferior.

In the biomaterial-derived sheet-like decellularized material as defined in accordance with the present invention, the elongation rate in the direction exhibiting the maximum tensile strength is 50% to 300%, preferably 100 to 250%, and more preferably 150 to 220%. By setting the elongation rate in this range, the artificial blood vessel prepared by rolling the biomaterial-derived sheet-like decellularized material has extremely high pressure resistance strength. On the other hand, as understood from Test Examples 3 and 4, in the biomaterial-derived sheet-like decellularized material prepared using a porcine aorta or bovine aorta, the elongation rates are respectively 304% and 332%, so that the pressure resistance strength of the artificial blood vessels prepared by rolling the same are inferior.

The biomaterial-derived sheet-like decellularized material has an anisotropy in the tensile strength and a maximum stress ratio of 1.5 to 5. In that case, it is preferable to roll the biomaterial-derived sheet-like decellularized material as defined in accordance with the present invention to prepare an artificial blood vessel, so as to increase the tensile strength in the circumferential direction of the artificial blood vessel.

An artificial blood vessel prepared by rolling a sheet-like decellularized material prepared using a porcine aorta or bovine aorta, which was conventionally considered to be optimum, has a low pressure resistance strength such as 16 KPa (120 mmHG) and 16.13 kPa (121 mmHg) as described in Test Examples 3 and 4. In contrast, an artificial blood vessel prepared by rolling the biomaterial-derived sheet-like decellularized material having a maximum value of tensile strength in four directions of 4 MPa or more and an elongation rate in the direction exhibiting the maximum tensile strength of 50% to 300% as defined in accordance with the present invention, has an extremely higher pressure resistance strength such as 1,000 mmHg or more compared with one prepared from a porcine aorta-derived material which was conventionally considered to be optimum, as described in Test Examples 3 and 4. Namely, the pressure resistance strength of the artificial blood vessel of the present invention is 1,000 mmHg or more.

2. Artificial blood vessel

An artificial blood vessel can be prepared by using the biomaterial-derived sheet-like decellularized material as defined in accordance with the present invention. A shape of the front cross-section of the artificial blood vessel of the present invention includes, for example, a circular shape, a substantially circular shape, an elliptical shape, a substantially elliptical shape, and the like. The shape is highly flexible, and may be deformed in accordance with the intended use. Generally, the inner circumference is preferably 1.5 to 200 mm, more preferably 3 to 70 mm, and still more preferably 6 to 40 mm.

In the artificial blood vessel of the present invention, it is preferable that a part of the wall portion forming the artificial blood vessel has a two-layer structure, from the viewpoint of pressure resistance. Embodiments where a part of the wall portion forming the artificial blood vessel has a two-layer structure include an artificial blood vessel having a two-layer structure and a three-layer structure as the wall portion (FIG. 1 (i)), an artificial blood vessel in which the entire wall portion is a two-layer structure, and an artificial blood vessel having a single-layer structure and a two-layer structure as the wall portion (FIG. 1 (ii)).

The length of the two-layer structure portion in the artificial blood vessel having a two-layer structure and a three-layer structure as the wall portion (the distance on the outer wall from point g on the outer wall clockwise up to point h, in FIG. 1 (i)) is preferably 0% or more, more preferably 50% or more, and still more preferably 80% or more of the length of the outer circumference. The length of the outer circumference denotes herein the length of the outer wall portion of the artificial blood vessel, taking one specific point of the artificial blood vessel as the starting point and end point. For example, the length of the outer circumference in FIG. 1 (i) denotes the length of the outer wall portion of the artificial blood vessel, taking point g as the starting point and end point.

The length of the two-layer structure portion in the artificial blood vessel having a single-layer structure and a two-layer structure as a wall portion (the distance on the outer wall from point j on the outer wall anticlockwise up to point k, in FIG. 1 (ii)) is preferably 10% or more, more preferably 50% or more, and still more preferably 80% or more of the length of the outer circumference. The length of the outer circumference denotes herein the length of the outer wall portion of the artificial blood vessel, taking one specific point of the artificial blood vessel as the starting point and end point. For example, the length of the outer circumference in FIG. 1 (ii) denotes the length of the outer wall portion of the artificial blood vessel, taking point j as the starting point and end point.

In the artificial blood vessel of the present invention, it is preferable to use a biomaterial-derived sheet-like decellularized material having a shape ("taper") in which the thickness of at least one side of the edge portion decreases toward the end of the edge portion as described in Patent Literature 2. Namely, both ends or one end of the cross-section of the biomaterial-derived sheet-like decellularized material have a tapered shape. The cross-section of the biomaterial-derived sheet-like decellularized material need not be processed linearly. The tapered portion may be provided at the edge portions on all four sides of the biomaterial-derived sheet-like decellularized material. The tapered portion may be provided at the edge portions on three sides, two sides or one side of the biomaterial-derived sheet-like decellularized material. The biomaterial-derived sheet-like decellularized material is rolled so that the tapered portion is on the inner lumen side of the artificial blood vessel, to form an artificial blood vessel. Preferably, the edge portions on at least two sides of the biomaterial-derived sheet-like decellularized material are tapered, and more preferably the edge portions on two sides of the biomaterial-derived sheet-like decellularized material in the length direction are tapered. Still more preferably, the edge portion on one side of the biomaterial-derived sheet-like decellularized material is tapered, and most preferably, the edge portion on one side in the length direction is tapered.

In one embodiment of preparation of the artificial blood vessel, the artificial blood vessel can be formed by rolling the biomaterial-derived sheet-like decellularized material on a core member. Concerning the outer circumference and the length of the core member, various types of core members can be selected, depending on the intended inner circumference and the length of the artificial blood vessel, and the quality of the material does not matter. The outer circumference of the core member to be used corresponds substantially to the inner circumference of the artificial blood vessel, and the core member may be appropriately selected in accordance with the intended inner circumference of the artificial blood vessel. The core material is not particularly limited, and examples thereof include a tube or cylindrical bar made of polytetrafluoroethylene (PTFE), polyurethane (PU) or a stainless steel material (SUS).

The artificial blood vessel of the present invention can be formed by stitching a part of the biomaterial-derived sheet-like decellularized material, or by bonding a part of the biomaterial-derived sheet-like decellularized material using an adhesive, or by resorting to both of the foregoing. Bonding with use of an adhesive is preferable from the viewpoint of processability. Therefore, the tapered portion of the biomaterial-derived sheet-like decellularized material may be fixed to the inner wall of the artificial blood vessel by means of, for example, stitching or an adhesive.

A conventionally used adhesive for biological tissue may be used herein as the adhesive to be used. Examples thereof include a fibrin glue, cyanoacrylate-based polymerizable adhesive, and gelatin glue resulting from cross-linking of gelatin and resorcinol by formalin, and the like, and a fibrin glue is preferable from the viewpoint of pressure resistance. A fibrin glue denotes herein a formulation in which pasty clots formed through the action of the enzyme thrombin on fibrinogen are utilized, for example, in tissue closure, adhesion in organ damage, hemostasis and the like.

The site at which the adhesive is applied is not particularly limited, as long as the adhesive allows bonding of the biomaterial-derived sheet-like decellularized material so as to form the artificial blood vessel. However, the adhesive is preferably applied so that no adhesive is present on the inner wall surface of the artificial blood vessel. This is because the adhesive may give rise to some adverse effects when coming into contact with substances passing through the inside (lumen) of the artificial blood vessel. Further, it is necessary to sufficiently apply the adhesive to the tapered portion and bond it so that the cross-section of the artificial blood vessel is circular, substantially circular, elliptical or substantially elliptical, as shown in FIGS. 1 (i) and (ii). This is because when bonding of the tapered portion is insufficient, pressure resistance at the affected portion may be impaired and the desired artificial blood vessel may not be obtained.

The artificial blood vessel of the present invention is used as an artificial blood vessel, but may also be used as a graft of a tubular biological tissue such as an ureter, trachea, and lymph duct.

The artificial blood vessel of the present invention has a much higher pressure resistance strength than one derived from a porcine aorta, which was conventionally considered to be optimum. The pressure resistance strength of the artificial blood vessel of the present invention is 133.3 kPa (1,000 mmHg) or more. In addition, the artificial blood vessel of the present invention exhibits excellent handleability during surgery or the like.

The biomaterial-derived sheet-like decellularized material as defined in accordance with the present invention may also be used to repair a blood vessel as a blood vessel repairing material. For example, the biomaterial-derived sheet-like decellularized material as defined in accordance with the present invention may be used for treatment such as application to a damaged part of a blood vessel.

EXAMPLE

Hereinafter, the present invention is explained in more detail with reference to examples, but the present invention is not limited only to these examples.

Example 1 (comparative)

Preparation of porcine aortic sheet-like decellularized material

An adventitia of a porcine aorta was completely stripped off, and was cut open to obtain a sheet-like aorta. The obtained sheet in a polyethylene zippered bag was subjected to a high hydrostatic pressure treatment for 15 minutes at 100 MPa in a high pressure processing device for research and development (Dr. CHEF, Kobe Steel, Ltd.), using a physiological saline as a medium. The treated sheet was shaken for 96 hours at 4 °C in a physiological saline containing 20 ppm of the nucleolytic enzyme DNase, followed by a treatment for 72 hours at 4 °C in 80% ethanol, and was lastly washed for 2 hours at 4 °C in a physiological saline, to obtain a porcine aortic sheet-like decellularized material.

Example 2 (comparative)

Preparation of porcine aortic decellularized artificial blood vessel

The porcine aortic sheet-like decellularized material prepared in Example 1 was cut and shaped to 24 mm × 100 mm. A biological adhesive fibrin glue was applied to the surface on the medial tissue side of the porcine aortic sheet-like decellularized material, and the porcine aortic sheet-like decellularized material was rolled twice on a core member of a PTFE tube having an outer diameter of 3.0 mm in such a manner that the intimal tissue was inside after formation of the artificial blood vessel, and the porcine aortic sheet-like decellularized material was pressed and shaped for 5 minutes. It was immersed in a physiological saline, and the PTFE tube of the core member was removed, and both ends were cut to prepare an artificial blood vessel of 100 mm × 3 mmΦ. Rolling and shaping were performed so that the flow path was formed in the same direction as the flow path of the aorta.

Test Example 1

Pressure resistance test

One end of a porcine aorta was clamped with forceps, and the opposite end was cannulated and ligated. A syringe and a manometer were connected to the cannula. A physiological saline in the syringe was injected into the porcine aorta, and the pressure at the burst of the porcine aorta was measured as a pressure resistance strength. The results are shown in Table 1.

One end of the artificial blood vessel prepared in Example 2 was clamped with forceps, and the opposite end was cannulated and ligated. A syringe and a manometer were connected to the cannula. A physiological saline in the syringe was injected into the artificial blood vessel, and the pressure at the burst of the artificial blood vessel was measured as a pressure resistance strength. The results are shown in Table 1.

[Table 1] mmHg corresponds to 13.33 kPa.

| Material | Pressure resistance strength (mmHg) |
|---|---|
| Porcine aorta | >1,000 |
| Porcine aortic decellularized rolled blood vessel | 120 |

Example 3

Preparation of porcine pericardial sheet-like decellularized material

A collected porcine pericardial sheet in a polyethylene zippered bag was subjected to a high hydrostatic pressure treatment for 15 minutes at 100 MPa in a high pressure processing device for research and development (Dr. CHEF, Kobe Steel, Ltd.), using a physiological saline as a medium. The treated sheet was shaken for 96 hours at 4 °C in a saline containing 20 ppm of the nucleolytic enzyme DNase, followed by a treatment for 72 hours at 4 °C in 80% ethanol, and was lastly washed for 2 hours at 4 °C in a physiological saline, to obtain a porcine pericardial sheet-like decellularized material.

Example 4 (comparative)

Preparation of porcine dermal sheet-like decellularized material

A dermal layer was separated from a porcine skin to obtain a sheet-like dermis. The sheet-like dermis and a physiological saline as a medium for a high hydrostatic pressure treatment were added to a polyethylene zippered bag. The mixture was pressurized at 100 MPa of a hydrostatic pressure for 15 minutes using a high pressure processing device for research and development (Dr. CHEF, Kobe Steel, Ltd.). The sheet-like dermis subjected by a high hydrostatic pressure treatment was shaken and washed for 96 hours at 4 °C in a physiological saline containing 20 ppm of the nucleolytic enzyme DNase, followed by a treatment for 72 hours at 4 °C in 80% ethanol, and was lastly washed for 2 hours at 4 °C in a physiological saline, to obtain a porcine dermal sheet-like decellularized material.

Test Example 2

Tensile test of porcine-derived sheet-like decellularized material

(1) Collection and preparation of test piece

Dumbbell test pieces of No. 8 described in ISO 37 were collected from the rectangular sheet-like decellularized materials prepared in Examples 1, 3 and 4 (porcine aorta, porcine pericardium, and porcine dermis). In order to evaluate the anisotropy of the tensile strength in the sheet, dumbbell test pieces were prepared from one sheet in the direction of 0°, 30°, 60° and 90°, assuming the direction is 0° in the case where a dumbbell test piece is prepared in the direction parallel to the long side.

(2) Measurement of test piece

The thickness of the parallel portion of the dumbbell test piece was measured using One-Shot 3D Measuring Macroscope (VR-3200, Keyence Corporation). The length (40 mm) between the cutting end faces of the punching blade of the parallel portion was used as the width (mm) of the test piece.

A cross-sectional area A (mm$^2$) of the test piece was calculated from the thickness and width of the test piece by the following equation.

$$A = t \times w$$

(A: cross sectional area of test piece ($mm^2$); t: thickness of test piece (mm); w: width of test piece (mm))

(3) Test procedure

The tensile test was performed in accordance with ISO 37, as follows. The test piece was attached to Table Top Universal Testing Machine (MCT-2150, A&D Company, Limited) so that both ends of the test piece were held symmetrically in order to uniformly distribute the tensile force to the cross section. The testing machine was operated to continuously observe changes in the distance between the marked lines and changes in the force, and the maximum load $F_{max}$ (N) and the distance between the marked lines at the breakage Lb (mm) were measured. The speed of the holding tool was set to 200 mm/min. Data of the test pieces broken outside of the marked lines were discarded. The test was repeated on additional test pieces until the measurements of the test pieces punched in four directions were made twice correctly for each direction. The tensile strength in four directions, the anisotropy of the tensile strength, and the elongation rate were calculated from the measured values by the following equations. The results are shown in Table 2.
The "tensile strength in four directions" and the "stress ratio" were compared among the four directions based on the average value in each direction.

(4) Calculation of results

<Tensile strength: σ>

σ (MPa ($N/mm^2$)) was calculated by the following equation.

$$\sigma = F_{max} / A$$

(σ: tensile strength (MPa); $F_{max}$: maximum load (N); A: cross-sectional area of test piece ($mm^2$))

<Elongation rate (elongation at the breakage): ε>

ε (%) was calculated by the following equation.

$$\varepsilon = (Lb - L0) / L0 \times 100$$

(Lb: distance between the marked lines at the breakage (mm); L0: initial distance between the marked lines (mm))

<Anisotropy in sheet>

Anisotropy in sheet was treated as a stress ratio S calculated by the following equation.

$$S = \sigma_{max} / \sigma_{min}$$

($\sigma_{max}$: the maximum tensile strength (MPa) in the tensile tests in four directions; $\sigma_{min}$: the minimum tensile strength (MPa) in the tensile tests in four directions)

Example 5

Preparation of porcine-derived decellularized artificial blood vessel

The sheet-like decellularized materials prepared in Examples 3 and 4 were cut and shaped to 24 mm × 100 mm. The surface water was wiped off. A biological adhesive fibrin glue was applied thereto, and the sheet-like decellularized materials were rolled twice on a core member of a PTFE tube having an outer diameter of 3.0 mm, and the sheet-like decellularized materials were pressed and shaped for 5 minutes. They were immersed in a physiological saline, and the PTFE tube of the core member was removed, and both ends were cut to prepare artificial blood vessels of 100 mm × 3 mmΦ. Rolling and shaping were performed so that the tensile strength in the circumferential direction of the artificial blood vessels increased.

Test Example 3

Tensile test and pressure resistance test of porcine-derived decellularized artificial blood vessel

One ends of three artificial blood vessels prepared in Examples 2 and 5 were clamped with forceps, and the opposite ends were cannulated and ligated. A syringe and a manometer were connected to the cannula. A physiological saline in the syringe was injected into the artificial blood vessel, and the pressure at the burst of the artificial blood vessel was measured as a pressure resistance strength. The results are shown in Table 2.

[Table 2] 1 mmHg corresponds to 13.33 kPa.

| | Before rolling | | | | | After rolling |
|---|---|---|---|---|---|---|
| | Thickness *1 (μm) | maximum value of tensile strength in four directions *2 (MPa) | S (stress ratio) | Elongation rate (%) | | Pressure resistance strength (mmHg) |
| | | | | Whole average | In direction exhibiting maxium tensile sterength | |
| Porcine aorta | 585 | 3.1 | 7.3 | 304 | 305 | 120 |
| Porcine dermis | 1,120 | 8.7 | 1.4 | 173 | 144 | 626 |
| Porcine pericardium | 349 | 5.9 | 2.1 | 158 | 170 | 1,347 |
| *1 Thickness is average of thickness of test pieces in four directions. *2 Maimum value of tensile strength is the maximum value in all tensile strengths mesured in four directions. | | | | | | |

Example 6

Preparation of bovine-derived sheet-like decellularized material and bovine-derived decellularized artificial blood vessel

A bovine aortic sheet-like decellularized material (comparative example), and a bovine pericardial sheet-like decellularized material, and a bovine aortic decellularized artificial blood vessel (comparative example) and a bovine pericardial decellularized artificial blood vessel were prepared in the same manner as in Examples 1 to 5 except that a bovine aorta was used in place of a porcine aorta and a bovine pericardium was used in place of a porcine pericardium.

Test Example 4

Tensile test and pressure resistance test of bovine-derived decellularized artificial blood vessel

The tensile test and the pressure resistance test were conducted in the same manner as in Test example 3, using the bovine-derived sheet-like decellularized materials and the bovine-derived decellularized artificial blood vessel prepared in Example 6. The results are shown in Table 3.

[Table 3] 1 mmHg corresponds to 13.33 kPa.

| | Before rolling | | | | | After rolling |
|---|---|---|---|---|---|---|
| | Thickness *1 ($\mu$m) | maximum value of tensile strength in four directions *2 (MPa) | S (stress ratio) | Elongation rate (%) | | Pressure resistance strength (mmHg) |
| | | | | Whole average | In direction exhibiting maxium tensile sterength | |
| Bovine aorta | 910 | 3.9 | 10.7 | 350 | 332 | 121 |
| Bovine pericardium | 648 | 12.7 | 2.0 | 184 | 206 | 1,063 |
| *1 Thickness is average of thickness of test pieces in four directions. *2 Maimum value of tensile strength is the maximum value in all tensile strengths mesured in four directions. | | | | | | |

The embodiments and examples disclosed herein are illustrative and non-restrictive in every aspect. The scope of the present invention is indicated by the claim and not by the above description.

INDUSTRIAL APPLICABILITY

The biomaterial-derived sheet-like decellularized material as defined in accordance with the present invention can provide an artificial blood vessel having a much higher pressure resistance strength than one derived from a porcine aorta, which was conventionally considered to be optimum. Therefore, when the biomaterial-derived sheet-like decellularized material as defined in accordance with the present invention is used as an artificial blood vessel or for repair of a blood vessel, excellent pressure resistance comparable to that of a blood vessel itself can be maintained.

## Claims

1.  An artificial blood vessel, a graft of a tubular biological tissue or a blood vessel repairing material, which comprises a biomaterial-derived sheet-like decellularized material,

    wherein the material is derived from pericardium, and has a maximum value of tensile strength in four directions of 4 MPa or more and an elongation rate in the direction exhibiting the maximum tensile strength of 50% to 300%, the tensile strength is anisotropic and a maximum stress ratio is 1.5 to 5,
    a pressure resistance strength of the artificial blood vessel, the graft of a tubular biological tissue or the blood vessel repairing material is 133.3 kPa (1000 mmHg) or more, and
    wherein the material is rolled to form the artificial blood vessel, the graft of a tubular biological tissue or the blood vessel repairing material, so as to provide increased tensile strength in the circumferential direction of the artificial blood vessel, the graft of a tubular biological tissue or the blood vessel repairing material.

## Patentansprüche

1.  Künstliches Blutgefäß, Transplantat eines röhrenförmigen biologischen Gewebes oder Blutgefäßreparaturmaterial, welches ein aus Biomaterial stammendes, plattenartiges dezellularisiertes Material umfasst,

    wobei das Material aus einem Herzbeutel stammt und einen Maximalwert der Zugfestigkeit in vier Richtungen von 4 MPa oder mehr aufweist und eine Dehnungsrate in der die maximale Zugfestigkeit aufweisenden Richtung von 50 % bis 300 % aufweist,
    die Zugfestigkeit anisotrop ist und ein maximales Spannungsverhältnis 1,5 bis 5 ist,
    eine Druckwiderstandsfestigkeit des künstlichen Blutgefäßes, Transplantats eines röhrenförmigen biologischen Gewebes oder des Blutgefäßreparaturmaterials 133,3 kPa (1000 mmHg) oder mehr ist, und
    wobei das Material zum Bilden des künstlichen Blutgefäßes, des Transplantats eines röhrenförmigen biologischen Gewebes oder des Blutgefäßreparaturmaterials gerollt ist, um eine erhöhte Zugfestigkeit in der Umfangsrichtung des künstlichen Blutgefäßes, des Transplantats eines röhrenförmigen biologischen Gewebes oder des Blutgefäßreparaturmaterials bereitzustellen.

**Revendications**

1. - Vaisseau sanguin artificiel, greffe d'un tissu biologique tubulaire ou matériau de réparation de vaisseau sanguin, qui comprend une matière décellularisée de type feuille, issue d'un biomatériau,

   dans lequel la matière est issue du péricarde et a une valeur maximale de résistance à la traction dans les quatre directions de 4 MPa ou plus et un taux d'allongement dans la direction présentant la résistance maximale à la traction de 50 % à 300 %;
   la résistance à la traction est anisotrope et un rapport de contrainte maximale est de 1,5 à 5 ;
   une force de résistance à la pression du vaisseau sanguin artificiel, de la greffe d'un tissu biologique tubulaire ou du matériau de réparation de vaisseau sanguin est de 133,3 kPa (1000 mmHg) ou plus ; et
   la matière est roulée pour former le vaisseau sanguin artificiel, la greffe d'un tissu biologique tubulaire ou le matériau de réparation de vaisseau sanguin, de façon à fournir une force de résistance à la traction accrue dans la direction périphérique du vaisseau sanguin artificiel, de la greffe d'un tissu biologique tubulaire ou du matériau de réparation de vaisseau sanguin.

FIG. 1

(i)

(ii)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016194895 A1
- WO 2014109185 A1
- WO 2016136633 A1
- WO 2014034759 A1
- WO 2014109185 A
- WO 2016194895 A

**Non-patent literature cited in the description**

- **E. PASQUINO et al.** *Journal of Materials Science: Materials in Medicine*, 01 January 1994, vol. 5, 850-854
- **S. JAHNAVI et al.** *Materials Science and Engineering*, 01 June 2015, vol. 51, 263-273